(19) Europäisches Patentamt
European Patent Office
Office européen des brevets

(11) Numéro de publication : **0 063 509**
**B1**

(12) **FASCICULE DE BREVET EUROPÉEN**

(45) Date de publication du fascicule du brevet : **20.02.85**

(21) Numéro de dépôt : **82400551.6**

(22) Date de dépôt : **26.03.82**

(51) Int. Cl.[4] : **C 07 D239/48**, C 07 D239/42, C 07 D239/47, C 07 D239/52, C 07 D239/54, C 07 D239/60, A 61 K 31/505

(54) **Nouveaux dérivés de pipérazino-2 pyrimidine, procédés pour leur préparation, médicaments les contenant et leur utilisation comme intermédiaires pour la fabrication de médicaments.**

(30) Priorité : **07.04.81 FR 8106924**

(43) Date de publication de la demande : **27.10.82 Bulletin 82/43**

(45) Mention de la délivrance du brevet : **20.02.85 Bulletin 85/08**

(84) Etats contractants désignés : **BE CH DE FR GB IT LI LU NL SE**

(56) Documents cités :
**DE-B- 1 620 419**
**FR-A- 2 173 746**
**FR-A- 2 257 294**
**FR-A- 2 281 117**
**FR-A- 2 311 776**
**US-A- 2 543 972**
**Le dossier contient des informations techniques présentées postérieurement au dépôt de la demande et ne figurant pas dans le présent fascicule.**

(73) Titulaire : **PHARMUKA LABORATOIRES**
**35 Quai du Moulin de Cage**
**F-92231 Gennevilliers (FR)**

(72) Inventeur : **Audiau, François**
**9, rue guérin**
**F-94220 Charenton (FR)**
Inventeur : **Gueremy, Claude Georges Alexandre**
**3, rue Daumesnil**
**F-78800 Houilles (FR)**
Inventeur : **Le Fur, Gérard Roger**
**35, rue du Progrès**
**F-92350 Plessis Robinson (FR)**

(74) Mandataire : **Gaumont, Robert et al**
**RHONE-POULENC RECHERCHES Service Brevets**
**Pharma 25, Quai Paul Doumer**
**F-92408 Courbevoie Cedex (FR)**

**EP 0 063 509 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention a pour objet de nouveaux dérivés de pipérazino-2 pyrimidine, leurs procédés de préparation et leur utilisation soit comme produits intermédiaires pour la fabrication de médicaments, soit comme médicaments antihypertenseurs, antimigraineux, antidépresseurs ou comme médicaments pour le traitement de la sénesence, de la maladie de Parkinson ou des symptômes observés lors du sevrage aux opiacés.

Il est déjà connu des dérivés d'amino-2 chloro-6 pipérazino-4 pyrimidine ayant des activités antiémétique, neuroleptique, analgésique, anti-sérotonine et spasmolytique [(cf. par exemple les brevets français 2 173 746, 2 257 294, 2 281 117 et G. Mattioda et Coll., J. Med. Chem. *18*, 553, (1975)].

Il est également connu du brevet FR 2 311 776 la N-méthylpipérazinyl-2 isopropylamino-4 méthylthio-5 chloro-6 pyrimidine comme produit intermédiaire pour la préparation de médicaments antiulcères.

Les composés selon l'invention peuvent être représentés par la formule générale

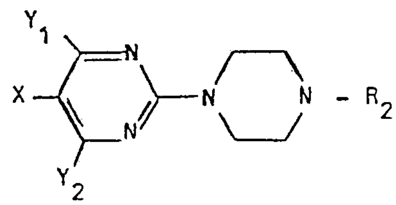

(I)

dans laquelle X est un atome d'hydrogène ou de chlore ou un groupe alkyle, alcoxy ou alkylthio ayant 1 à 3 atomes de carbone, $R_2$ est un atome d'hydrogène ou un groupe alkyle ayant 1 à 3 atomes de carbone, $Y_2$ est un atome de chlore et $Y_1$ est un atome de chlore ou un groupe

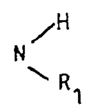

dans lequel $R_1$ est un atome d'hydrogène, un groupe alkyle à chaîne droite ou ramifiée ayant 1 à 7 atomes de carbone, un groupe cycloalkyle ayant 3 à 7 atomes de carbone ou un groupe cycloalkylalkyle ayant 4 à 8 atomes de carbone, ou bien $Y_2$ et $Y_1$ sont tous les deux des groupes OH, à l'exception de la N-méthylpipérazinyl-2 isopropylamino-4 méthylthio-5 chloro-6 pyrimidine.

Dans la formule (I) ci-dessus, quand $Y_1$ est un groupe

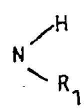

$R_1$ est de préférence un groupe alkyle à chaîne ramifiée, en particulier un groupe isopropyle.

Les composés de formule (I) pour lesquels $Y_1$ et $Y_2$ sont des groupes OH peuvent être préparés par condensation d'un diester de formule

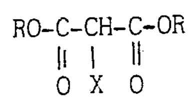

(II)

dans laquelle X à la même signification que dans la formule (I) et R est un groupe alkyle de bas poids moléculaire tel que méthyle ou éthyle, avec une amidine de formule

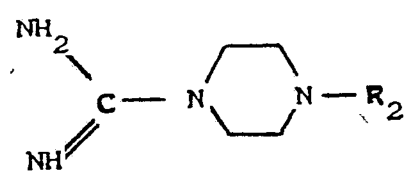

(III)

dans laquelle $R_2$ a la même signification que dans la formule (I), selon le schéma réactionnel suivant

(a)

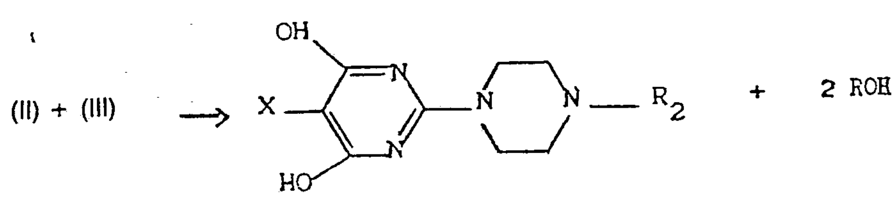

(II) + (III)

+ 2 ROH

La réaction de condensation (a) ci-dessus peut être effectuée selon des procédés connus en soi (cf par exemple The Pyrimidines, Interscience Publishers, p. 51, 1962). Elle peut être réalisée en particulier au sein du méthanol, à la température de reflux du solvant et en présence de méthylate de sodium.

Les composés de formule (I) pour lesquels $Y_1$ et $Y_2$ sont des atomes de chlore peuvent être préparés par action d'un agent chlororant sur les composés de formule (I) pour lesquels $Y_1$ et $Y_2$ sont des groupes OH. Cette réaction peut être effectuée selon des procédés connus en soi (cf. The Pyrimidines, Interscience Publischers, p. 51, 1962). Comme agent chlorurant utilisable, on peut citer en particulier l'oxychlorure de phosphore.

Les composés de formule (I) pour lesquels $Y_1$ et $Y_2$ sont des atomes de chlore et $R_2$ est un groupe alkyle ayant 1 à 3 atomes de carbone peuvent aussi être préparés par condensation d'une trichloro-2,4,6 pyrimidine de formule (IV), dans laquelle X a la même signification que dans la formule (I), avec une pipérazine de formule (V) dans laquelle $R_2$ est un groupe alkyle ayant 1 à 3 atomes de carbone, selon le schéma réactionnel suivant

(b) 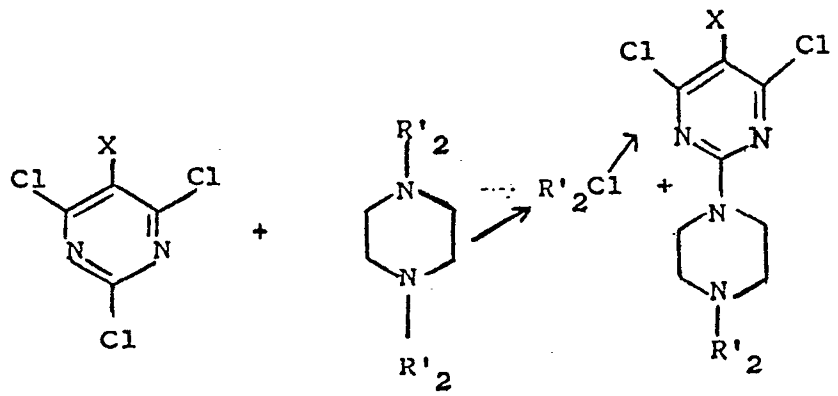

(IV) (V)

La réaction de condensation (b) est effectuée selon des procédés connus en soi [cf The Pyrimidines, p. 345, (1962) ; idem, supplément I, p. 133, (1970)]. Elle peut, par exemple, être réalisée en chauffant au sein d'un solvant inerte tel que hydrocarbure aromatique (par exemple le toluène ou le xylène), à une température comprise entre 50 °C et 150 °C, les composés (IV) et (V).

Les composés de formule (I) pour lesquels $Y_1$ est un groupe

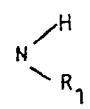

$Y_2$ est un atome de chlore et $R_2$ est un atome d'hydrogène peuvent être préparés par hydrolyse des dérivés N-acylés de formule

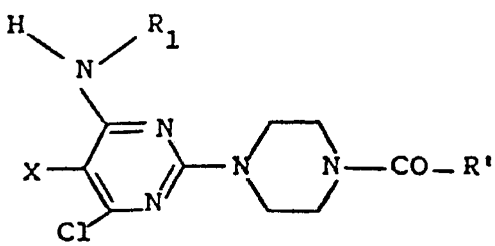 (VI)

dans laquelle X et $R_1$ ont le même signification que dans le formule (I) et R' est un atome d'hydrogène, un groupe alkyle de bas poids moléculaire (par exemple méthyle) ou le groupe phényle. Cette hydrolyse peut être réalisée en chauffant le composé (VI) au sein d'une solution d'un acide minéral tel que l'acide chlorhydrique ou l'acide sulfurique dans l'eau ou dans un mélange $H_2O$+ solvant miscible à l'eau (par exemple le méthanol, l'éthanol, l'acide acétique, le dioxanne), à la température de reflux du milieu.

Les dérivés N-acylés de formule (VI) peuvent être préparés par condensation d'une trichloro-2,4,6 pyrimidine de formule (IV) avec une pipérazine de formule (VII) et condensation du composé de formule (VIII) ainsi obtenu avec un composé de formule $R_1NH_2$, dans laquelle $R_1$ a la même signification que dans la formule (I), selon le schéma réactionnel suivant

(c)

(IV)

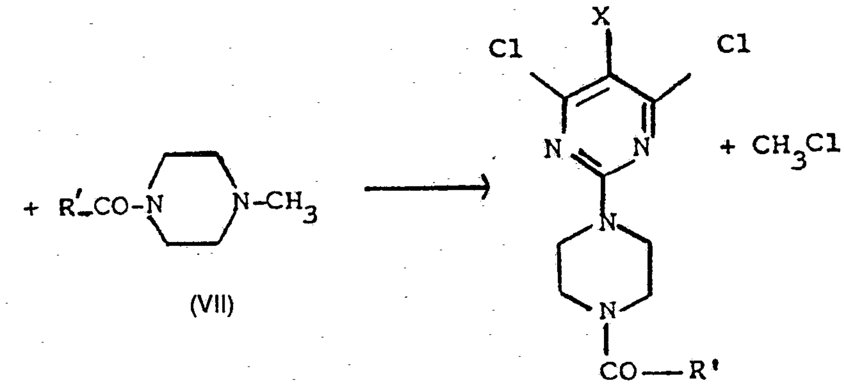

(VII)

(VIII)

(d) (VIII) + $R_1NH_2$ → (VI) + HCl

La réaction de condensation (c) est effectuée en chauffant les composés (IV) et (VII) au sein d'un solvant inerte tel qu'un hydrocarbure aromatique (par exemple le toluène ou le xylène) à une température comprise entre 50 °C et 150 °C. La réaction de condensation (d) est effectuée dans les mêmes conditions que la réaction de condensation(e).

Les composés de formule (I) pour lesquels $Y_1$ est un groupe

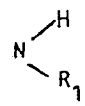

$Y_2$ est un atome de chlore et $R_2$ est un groupe alkyle peuvent être préparés par condensation d'un composé de formule $R_1NH_2$, dans laquelle $R_1$ a la même signification que dans la formule (I), avec un composé de formule (IX), dans laquelle X a la même signification que dans la formule (I) et $R'_2$ est un groupe alkyle ayant 1 à 3 atomes de carbone, selon le schéma réactionnel suivant :

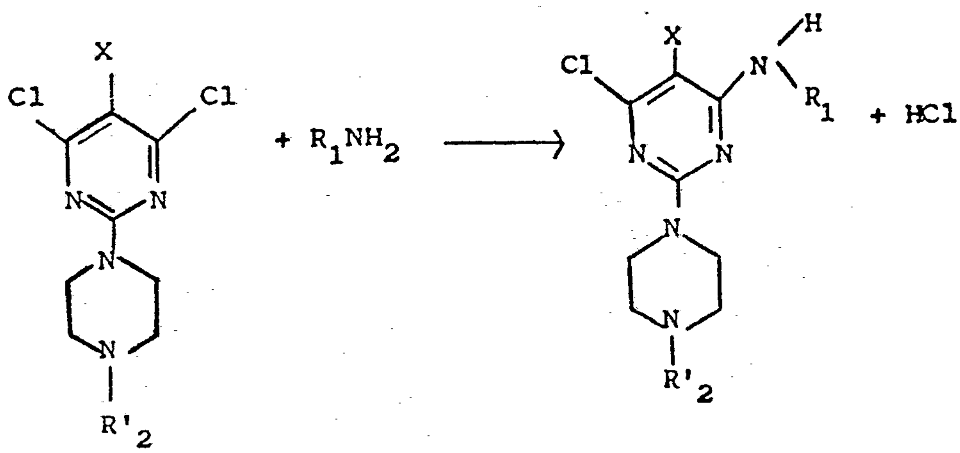

(e)

(IX)

La réaction de condensation (e) est effectuée en chauffant le composé (IX) et le composé $R_1NH_2$, à une température comprise entre 80 °C et 150 °C, au sein d'un solvant et de préférence en présence d'une base pour fixer l'acide chlorhydrique libéré. Comme solvants utilisables on peut citer des solvants inertes tels que des hydrocarbures (par exemple le toluène, le xylène), des alcools (par exemple le méthanol, l'éthanol), des cétones (par exemple la méthyléthylcétone) et des solvants aprotiques polaires (par exemple le diméthylformamide), ou un excès du composé $R_1NH_2$ lorsque celui-ci est une amine. Comme bases utilisables, on peut citer des bases minérales tells que le carbonate de potassium ou un excès du composé $R_1NH_2$, lorsque celui-ci est l'ammoniac, et des bases organiques telles qu'un excès du composé $R_1NH_2$, lorsque celui-ci est une amine. Selon le solvant et la température utilisés, la réaction du condensation (e) est effectuée sous une pression égale ou supérieure à la pression atmosphérique.

Les composés de formule (I) pour lesquels $Y_1$ est un groupe

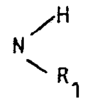

$Y_2$ est un atome de chlore et $R_2$ est un groupe alkyle peuvent aussi être préparés par condensation d'une pipérazine de formule (X), dans laquelle $R'_2$ est un groupe alkyle ayant 1 à 3 atomes de carbone, avec une dichloro-2,6 pyrimidine de formule (XI), dans laquelle X et $R_1$ ont la même signification que dans la formule (I), selon le schéma réactionnel suivant

(f)

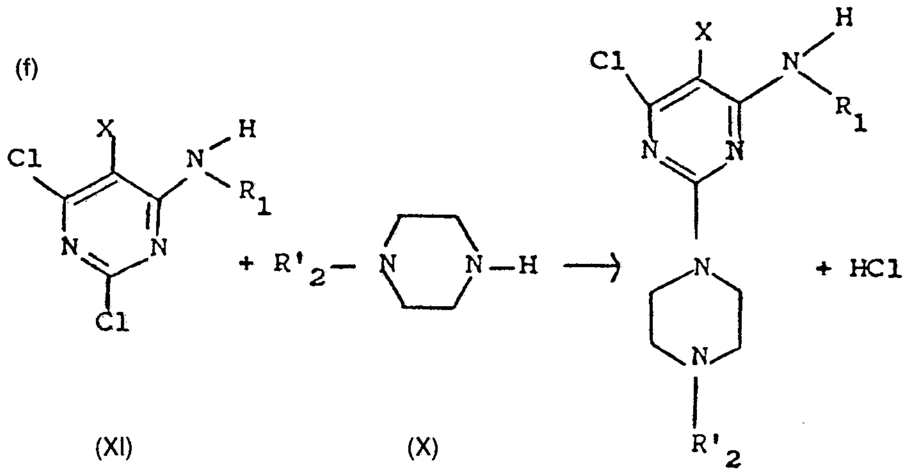

(XI) (X)

La réaction de condensation (f) est réalisée dans des conditions identiques à celles utilisées pour la réaction de condensation (e).

Les dichloro-2,6 pyrimidines de formule XI sont obtenues par condensation d'un composé de formule $R_1NH_2$, dans laquelle $R_1$ a la même signification que dans la formule (I), avec une trichloro-2,4,6 pyrimidine de formule (IV). Cette condensation est effectuée au sein d'un solvant inerte tel que ceux utilisés pour la réaction (e), par exemple la méthyléthylcétone, en présence d'une base minérale ou organique, par exemple la triéthylamine, à une température voisine de la température ambiante. Elle conduit en général à un mélange de deux isomères de position, comme l'indique le schéma réactionnel ci-dessous ;

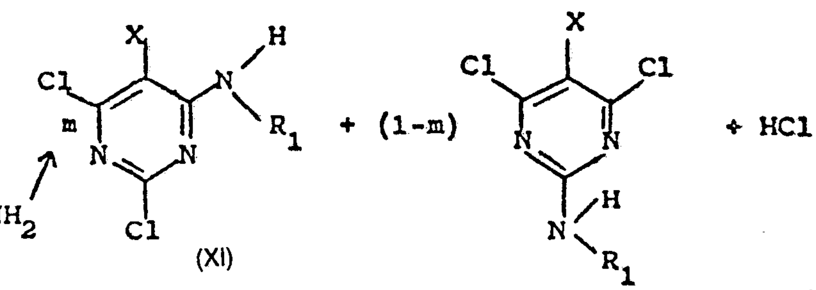

(IV) + $R_1NH_2$

(XI)

Ces deux isomères peuvent être séparés à partir de leur mélange par des méthodes classiques telles que la xhromatographie sur colonne de silice ou la recristallisation dans un solvant approprié.

Toutefois, dans le cas où X est le groupe méthoxy et à condition d'opérer dans les conditions définies ci-dessus (solvant : méthyléthylcétone ; base : triéthylamine ; température voisine de la température ambiante), la condensation de (IV) avec $R_1NH_2$ conduit pratiquement uniquement à l'isomère recherché, c'est-à-dire le composé (XI). Le procédé de synthèse des composés de formule (I) mettant en oeuvre la réaction (f) est donc particulièrement intéressant dans le cadre où $X = OCH_3$.

Les mélanges réactionnels obtenus par les divers procédés décrits précédemment sont traités suivant des méthodes classiques, physiques (évaporation , extraction à l'aide d'un solvant, distillation, cristallisation, chromatographie, etc...) ou chimiques (formation de sel et régénération de la base, etc...) afin d'isoler les composés (I) à l'état pur.

Les composés de formule (I) sous forme de base libre peuvent éventuellement être transformés en sels d'addition avec un acide minéral ou organique par action d'un tel acide au sein d'un solvant approprié.

Les composés de formule (I) pour lesquels $Y_2$ est un atome de chlore et $Y_1$ est un groupe

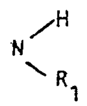

possèdent des propriétés pharmacologiques. Ils sont dénués d'activité antiémétique ou neuroleptique, mais ont la propriété de se lier aux récepteurs α de la noradrénaline. Bien qu'ayant une structure chimique éloignée de celle de la clonidine, ils déplacent cette dernière de ses sites de liaison. Or la clodinine se comporte comme un agoniste vis-à-vis des récepteurs α adrénergiques. En particulier elle présente une forte affinité pour la catégorie dénommée $\alpha_2$ de ces récepteurs, catégorie qui module entre autres la libération de noradrénaline au niveau central et au niveau périphérique.

Les exemples suivants illustrent l'invention sans la limiter.

## Exemple 1

N-méthylpipérazino-2 dihydroxy-4,6 pyrimidine

On chauffe au reflux, pendant 30 minutes, 47,7 g de sulfate acide de (méthyl-1 pipérazino-4) carboxamidine et une solution de méthylate obtenue par action de 11,5 g de sodium sur 270 ml de méthanol. Puis on ajoute 40 g de malonate de diéthyle et chauffe au reflux pendant 5 heures. Après refroidissement, on verse le mélange réactionnel dans l'eau, neutralise à l'acide acétique et évapore sous pression réduite. On reprend le résidu avec 1 l de chloroforme, chauffe à l'ébullition la suspension ainsi obtenue, puis refroidit. L'insoluble est essoré et séché sous pression réduite. On obtient ainsi 70 g d'un solide qui est constitué essentiellement de N-méthylpipérazino-2 dihydroxy-4,6 pyrimidine.

La (méthyl-1 pipérazino-4) carboxamidine est préparée comme indiqué par Stankevicius et Coll., Khim. Farm. Zh. *5* (1), 13-16 (1971).

## Exemple 2

N-méthylpipérazino-2 dichloro-4,6 pyrimidine.

La N-méthylpipérazino-2 dihydroxy-4,6 pyrimidine obtenue à l'exemple 1 est mise à réagir avec 180 ml d'oxychlorure de phosphore, d'abord à la température ambiante, puis à la température de reflux pendant 3 heures. Le mélange réactionnel est ensuite versé sur de la glace et on neutralise par addition d'hydroxyde de sodium. On extrait avec du chloroforme et la phase chloroforme est évaporée sous pression réduite. Le résidu obtenu est fixé sur une colonne de gel de silice et on élue avec un mélange toluène-diéthylamine 9/1 (9 parties en volume de toluène pour 1 partie en volume de diéthylamine). On obtient ainsi 8,4 g de N-méthylpipérazino-2 dichloro-4,6 pyrimidine.

## Exemple 3

N-méthylpipérazino-2 méthylamine-4 chloro-6 pyrimidine

On chauffe au reflux, pendant 32 heures, un mélange de 7 g de N-méthylpipérazino-2 dichloro-4,6 pyrimidine, 140 ml de toluène, 30 ml d'une solution éthanolique de méthylamine à 33 % en poids de méthylamine et 2,1 g de carbonate de potassium finement broyé. Puis on filtre, évapore le filtrat sous pression réduite, fixe le résidu sur une colonne de gel de silice et élue avec un mélange toluène-diéthylamine 9/1. Les fractions contenant le produit recherché sont évaporées et le résidu est dissous dans de l'oxyde diéthylique. On ajoute une solution d'acide chlorhydrique dans l'oxyde diéthylique. Le précipite formé est recristallisé dans l'éthanol. On obtient ainsi 4,8 g de dichlorhydrate de N-méthylpipérazino-2 méthylamino-4 chloro-6 pyrimidine, dont le point de fusion est supérieur à 260 °C.

## Exemple 4

N-méthylpipérazino-2 isopropylamino-4 chloro-6 pyrimidine

En remplaçant dans l'exemple 3 la méthylamine par l'isopropylamine, on obtient le chlorhydrate de N-méthylpipérazino-2 isopropylamino-4 chloro-6 pyrimidine, qui fond avec décomposition vers 225 °C.

## Exemple 5

N-méthylpipérazino-2 cyclopentylamino-4 chloro-6 pyrimidine

En remplaçant dans l'exemple 3 la méthylamine par la cyclpentylamine, on obtient le chlorhydrate de N-méthylpipérazino-2 cyclopentylamino-4 chloro-6 pyrimidine, qui fond à 264 °C.

Exemple 6

N-méthylpipérazino-2 chloro-5 dihydroxy-4,6 pyrimidine

On chauffe au reflux, pendant 30 mn, 57,3 g de sulfate acide de (méthyl-1 pipérazino-4) carboxamidine et une solution de méthylate obtenue par action de 13,8 g de sodium sur 320 ml de méthanol. On ajoute ensuite 61,2 g de chloro-malonate de diéthyle et on maintient 5 heures au reflux. Après refroidissement, on verse le mélange réactionnel dans l'eau, neutralise à l'acide acétique et évapore sous pression réduite. On reprend le résidu avec 500 ml de méthanol chaud, essore aussitôt l'insoluble, le lave avec 100 ml de méthanol et le sèche sous pression réduite. On obtient ainsi 77 g d'un produit solide constitué principalement de N-méthylpipérazino-2 chloro-5 dihydroxy-4,6 pyrimidine.

Exemple 7

N-méthylpipérazino-2 trichloro-4,5,6 pyrimidine

La N-méthylpipérazino-2 chloro-5 dihydroxy-4,6 pyrimidine obtenue à l'exemple 6 est mise à réagir avec 300 ml d'oxychlorure de phosphore, d'abord à la température ambiante, puis à la température de reflux pendant 5 heures. Le mélange réactionnel est ensuite versé sur de la glace et on neutralise par addition d'ammoniaque. On extrait avec du chloroforme et évapore la phase chloroformique sous pression réduite. On obtient ainsi 27,6 g de N-méthylpipérazino-2 trichloro-4,5,6 pyrimidine brute.

Exemple 8

N-méthylpipérazino-2 amino-4 dichloro-5,6 pyrimidine

On chauffe pendant 3 heures à 130 °C, en autoclave, 22 g de N-méthylpipérazino-2 trichloro-4,5,6 pyrimidine brute obtenue comme indiqué à l'exemple 7 et une solution de 42 g d'ammoniac dans 250 ml de méthanol. Puis on évapore sous pression réduite, fixe le résidu sur une colonne de gel de silice et élue avec un mélange chloroforme-diéthylamine 95/5. On obtient ainsi 11,3 g de N-méthylpipérazino-2 amino-4 dichloro-5,6 pyrimidine, dont le chlorhydrate fond à 260 °C.

Exemple 9

N-méthylpipérazino-2 méthylamino-4 dichloro-5,6 pyrimidine

On chauffe pendant 8 heures au reflux un mélange de 14 g de N-méthylpipérazino-2 trichloro-4,5,6 pyrimidine, 250 ml de toluène, 10 ml d'une solution alcoolique de méthylamine à 33 % en poids de méthylamine et 3,5 g de carbonate de potassium. Puis on lave à l'eau le mélange réactionnel et l'évapore sous pression réduite. Le résidu obtenu est fixé sur une colonne de gel de silice et on élue avec un mélange toluène-diéthylamine 9/1. On obtient ainsi 11,9 g de N-méthylpipérazino-2 méthylamino-4 dichloro-5,6 pyrimidine, dont le chlorhydrate fond au-dessus de 260 °C.

Exemple 10

N-méthylpipérazino-2 isopropylamino-4 dichloro-5,6 pyrimidine.

On opère comme à l'exemple 9, en partant de 10 g de N-méthylpipérazino-2 trichloro-4,5,6 pyrimidine au lieu de 14 g, et de 7,2 ml d'isopropylamine au lieu de la solution de méthylamine. On obtient 8,8 g de N-méthylpipérazino-2 isopropylamino-4 dichloro-5,6 pyrimidine dont le chlorhydrate fond à 252 °C.

Exemple 11

N-méthylpipérazino-2 cyclopentylamino-4 dichloro-5,6 pyrimidine

On opère comme à l'exemple 9, en partant de 22,5 g de N-méthylpipérazino-2 trichloro-4,5,6 pyrimidine au lieu de 14 g et de 25 ml de cyclopentylamine au lieu de la solution de méthylamine. On obtient 13,8 g de N-méthylpipérazino-2 cyclopentylamino-4 dichloro-5,6 pyrimidine, dont le chlorhydrate fond à 258 °C.

Exemple 12

N-méthylpipérazino-2 isopropylamino-4 méthoxy-5 chloro-6 pyrimidine

On agite à 20 °C, pendant 6 heures, 42 ml de triéthylamine, 25 ml d'isopropylamine, 250 ml de

méthyléthylcétone et 32 g de trichloro-2,4,6 méthoxy-5 pyrimidine [préparée comme indiqué par Budesinsky et Coll., Ceskoslov. Farm. *10*, 241-247, (1961)]. Le mélange réactionnel est ensuite évaporé sous pression réduite et le résidu est fixé sur une colonne de gel de silice. On élue avec un mélange toluène-diétrhylamine 95/5. On isole ainsi 20,2 g d'isopropylamino-4 dichloro-2,6 méthoxy-5 pyrimidine.

On chauffe au reflux, pendant 8 heures, un mélange de 12 g d'isopropylamino-4 dichloro-2,6 méthoxy-5 pyrimidine, 250 ml de toluène, 12 ml de N-méthylpipérazine et 3,5 g de carbonate de potassium. Puis on lave le mélange réactionnel à l'eau et évapore la phase organique sous pression réduite. Le résidu obtenu est fixé sur une colonne de gel de silice et on élue avec un mélange toluène-diéthylamine 9/1. On obtient ainsi 10,6 g de N-méthylpipérazino-2 isopropylamino-4 méthoxy-5 chloro-6 pyrimidine, dont le chlorhydrate fond à 142 °C.

Exemple 13

N-méthylpipérazino-2 dihydroxy-4,6 méthoxy-5 pyrimidine

On opère comme à l'exemple 6 en partant de 57,3 g de sulfate acide de (méthyl-1 pipérazino-4) carboxamidine et de 48,6 g de méthoxymalonate de diméthyle. On obtient ainsi la N-méthylpipérazino-2 dihydroxy-4,6 méthoxy-5 pyrimidine.

Exemple 14

N-méthylpipérazino-2 dichloro-4,6 méthoxy-5 pyrimidine

On opère comme à l'exemple 7 en remplaçant au départ la N-méthylpipérazino-2 chloro-5 dihydroxy-4,6 pyrimidine par la N-méthylpipérazino-2 méthoxy-5 dihydroxy-4,6 pyrimidine obtenue précédemment. On obtient 20,9 g de N-méthylpipérazino-2 dichloro-4,6 méthoxy-5 pyrimidine.

Exemple 15

N-méthylpipérazino-2 cyclopentylamino-4 méthoxy-5 chloro-6 pyrimidine

On chauffe à reflux, pendant 8 heures, 19,4 g de N-méthylpipérazino-2 dichloro-4,6 méthoxy-5 pyrimidine, 300 ml de toluène, 16 ml de cyclopentylamine et 11 g de carbonate de potassium. On lave à l'eau le mélange réactionnel et l'évapore sous pression réduite. Le résidu obtenu est fixé sur une colonne de gel de silice et on élue avec un mélange toluène-méthanol 85/15. On obtient ainsi 13,3 g de N-méthylpipérazino-2 cyclopentylamino-4 méthoxy-5 chloro-6 pyrimidine, dont le chlorhydrate fond au-dessus de 260 °C.

Exemple 16

N-méthylpipérazino-2 éthoxy-5 dihydroxy-4,6 pyrimidine

On opère comme à l'exemple 6 en partant de 96 g de sulfate acide de (méthyl-1 pipérazino-4) carboxamidine et de 102 g d'éthoxymalonate de diéthyle. On obtient ainsi la N-méthylpipérazino-2 éthoxy-5 dihydroxy 4,6 pyrimidine.

Exemple 17

N-méthylpipérazino-2 éthoxy-5 dichloro 4,6 pyrimidine

On opère comme à l'exemple 7 en remplaçant au départ la N-méthylpipérazino-2 chloro-5 dihydroxy-4,6 pyrimidine par la N-méthylpipérazino-2 éthoxy-5 dihydroxy-4,6 pyrimidine obtenue précédemment. On obtient 28 g de N-méthylpipérazino-2 dichloro-4,6 éthoxy-5 pyrimidine.

Exemple 18

N-méthylpipérazino-2 isopropylamino-4 éthoxy-5 chloro-6 pyrimidine

On chauffe au reflux, pendant 26 heures, 14,55 g de N-méthylpipérazino-2 éthoxy-5 dichloro-4,6 pyrimidine, 250 ml de méthyléthylcétone et 12,7 ml d'isopropylamine. Puis le mélange réactionnel est évaporé sous pression réduite et le résidu obtenu est fixé sur une colonne de gel de silice. On élue avec un mélange chloroforme-méthanol 95/5. On obtient ainsi 11,4 g de N-méthylpipérazino-2 isopropylamino-4 éthoxy-5 chloro-6 pyrimidine, dont le chlorhydrate fond à 174 °C.

Exemple 19

N-méthylpipérazino-2 méthyl-5 dihydroxy-4,6 pyrimidine

On opère comme à l'exemple 6 en partant de 96 g de sulfate acide de (méthyl-1 pipérazino-4) carboxamidine et de 87 g de méthylmalonate de diéthyle. On obtient ainsi la N-méthylpipérazino-2 méthyl-5 dihydroxy-4,6 pyrimidine.

Exemple 20

N-méthylpipérazino-2 méthyl-5 dichloro-4,6 pyrimidine

On opère comme à l'exemple 7 en remplaçant au départ la N-méthylpipérazino-2 chloro-5 dihydroxy-4,6 pyrimidine par la N-méthylpipérazino-2 méthyl-5 dihydroxy-4,6 pyrimidine obtenue précédemment. On obtient 97,8 g de N-méthylpipérazino-2 méthyl-5 dichloro-4,6 pyrimidine.

Exemple 21

N-méthylpipérazino-2 isopropylamino-4 méthyl-5 chloro-6 pyrimidine

On chauffe au reflux, pendant 48 h, 21 g de N-méthylpipérazino-2 dichloro-4,6 méthyl-5 pyrimidine, 300 ml de méthyléthylcétone et 20,4 ml d'isopropylamine. Le mélange réactionnel est évaporé sous pression réduite et le résidu est fixé sur une colonne de gel de silice. On élue avec un mélange chloroforme-méthanol 95/5. On obtient ainsi 9,1 g de N-méthylpipérazino-2 isopropylamino-4 méthyl-5 chloro-6 pyrimidine, dont le dichlorhydrate fond à 258 °C.

Exemple 22

N-méthylpipérazino-2 cyclopentylamino-4 méthyl-5 chloro-6 pyrimidine

On chauffe au reflux, pendant 48 h, 13 g de N-méthylpipérazino-2 méthyl-5 dichloro-4,6 pyrimidine, 250 ml de toluène, 11,2 ml de cyclopentylamine et 7,7 g de carbonate de potassium. Le mélange réactionnel est lavé à l'eau, puis évaporé sous pression réduite. Le résidu obtenu est fixé sur une colonne de gel de silice et on élue avec un mélange toluène-méthanol 85/15. On obtient ainsi 14,2 g de N-méthylpipérazino-2 cyclopentylamino-4 méthyl-5 chloro-6 pyrimidine, dont le chlorhydrate fond à 260 °C.

Exemple 23

N-méthylpipérazino-2 éthyl-5 dihydroxy-4,6 pyrimidine

On opère comme à l'exemple 6 en partant de 96 g de sulfate acide de (méthyl-1 pipérazino-4) carboxamidine et de 99 g d'éthylmalonate de diéthyle. On obtient ainsi la N-méthylpipérazino-2 éthyl-5 dihydroxy-4,6 pyrimidine.

Exemple 24

N-méthylpipérazino-2 éthyl-5 dichloro-4,6 pyrimidine

On opère comme à l'exemple 7 en remplaçant au départ la N-méthylpipérazino-2 chloro-5 dihydroxy-4,6 pyrimidine par la N-méthylpipérazino-2 éthyl-5 dihydroxy-4,6 pyrimidine obtenu précédemment. On obtient 67,6 g de N-méthylpipérazino-2 éthyl-5 dichloro-4,6 pyrimidine.

Exemple 25

N-méthylpipérazino-2 isopropylamino-4 éthyl-5 chloro-6 pyrimidine

On opère comme à l'exemple 21 en partant de 22 g de N-méthylpipérazine-2 éthyl-5 dichloro-4,6 pyrimidine, 300 ml de méthyléthylcétone et 20,4 ml d'isopropylamine. On obtient 6,7 g de N-méthylpipérazino-2 isopropylamino-4 éthyl-5 chloro-6 pyrimidine, dont le chlorhydrate fond à 198 °C.

Exemple 26

Pipérazino-2 isopropylamino-4 méthyl-5 chloro-6 pyrimidine

1. A une solution de 32 g de méthyl-1 formyl-4 pipérazine [préparée comme indiqué par J. Pharm. Soc. Japan, *74*, 1049-1051 (1954)] dans 250 ml de toluène, maintenue à 80 °C, on ajoute une solution de 49,4 g de trichloro-2,4,6 méthyl-5 pyrimidine [préparée comme indiqué dans Pharm. Bull. 1, 387-390 (1953)] dans 250 ml de toluène. Le mélange réactionnel est chauffé 4 heures à la température de 80 °C-85 °C, puis 2 heures au reflux. On filtre, évapore le filtrat sous pression réduite et fixe le résidu sur une

colonne de gel de silice. On élue avec un mélange toluène-méthanol 9/1 et obtient ainsi 26,3 g de N-formylpipérazino-2 dichloro-4,6 méthyl-5 pyrimidine.

2. Aux 26,3 g de ce dernier produit on ajoute 250 ml de toluène et 30 ml d'isopropylamine et on chauffe au reflux pendant 48 heures. On lave à l'eau la phase organique, puis l'évapore sous pression réduite. Le résidu est fixé sur une colonne de gel de silice et on élue avec un mélange toluène-méthanol 95/5. On obtient ainsi 14 g de N-formylpipérazino-2 isopropylamino-4 méthyl-5 chloro-6 pyrimidine, qui fond à 164 °C.

3. On chauffe au reflux, pendant 2 heures, 14 g de N-formylpipérazino-2 isopropylamino-4 méthyl-5 chloro-6 pyrimidine, 400 ml d'eau et 40 ml d'acide chlorhydrique concentré. On traite ensuite la solution avec 2 g de noir animal, filtre, alcalinise par addition d'hydroxyde de sodium et extrait avec du chloroforme. La phase chloroforme est évaporée sous pression réduite et le résidu est dissous dans l'éthanol. On ajoute une solution d'acide chlorhydrique dans l'éthanol. Le précipité formé est recristallisé deux fois dans l'eau. On obtient ainsi 6,4 g de chlorhydrate de pipérazino-2 isopropylamino-4 méthyl-5 chloro-6 pyrimidine, qui fond à 199 °C.

## Exemple 27

Pipérazino-2 isopropylamino-4 méthylthio-5 chloro-6 pyrimidine

1. En opérant comme dans la première partie de l'exemple 26 à partir de 42,3 g de méthyl-1 formyl-4 pipérazine et de 69 g de trichloro-2,4,6 méthylthio-5 pyrimidine, on obtient 94,3 g de N-formylpipérazino-2 dichloro-4,6 méthylthio-5 pyrimidine.

2. Aux 94,3 g de ce dernier produit on ajoute 700 ml de toluène, 52 ml de triéthylamine et 30 ml d'isopropylamine, et on chauffe à 100 °C pendant 4 heures. On filtre à chaud, lave le filtrat à l'eau et le concentre par distillation sous pression réduite. Le résidu est fixé sur une colonne de gel de silice et on élue avec un mélange toluène-diéthylamine 95/5. On obtient ainsi 86 g de N-formylpipérazino-2 isopropylamino-4 méthylthio-5 chloro-6 pyrimidine. Ce produit pésente, après deux recristallisations dans un mélange eau-éthanol 50/50, un point de fusion de 92 °C.

3. On opère comme à la troisième partie de l'exemple 26 en remplaçant les 14 g de N-formylpipérazino-2 isopropylamino-4 méthyl-5 chloro-6 pyrimidine par 80 g de N-formylpipérazino-2 isopropylamino-4 méthylthio-5 chloro-6 pyrimidine. APrès trois recristallisations dans l'eau du chlorhydrate formé au sein de l'éthanol, on obtient 30,5 g de chlorhydrate de pipérazino-2 isopropylamino-4 méthylthio-5 chloro-6 pyrimidine, qui fond à 147 °C.

## Exemple 28

N-méthylpipérazino-2 dichloro-4,6 méthylthio-5 pyrimidine

A une solution de 38 g de N,N'-diméthylpipérazine dans 300 ml de toluène on ajoute, en l'espace d'une heure, à la température d'environ 100 °C, une solution de 69 g de trichloro-2,4,6 méthylthio-5 pyrimidine dans 300 ml de toluène. On chauffe 2 heures à 90 °C, puis refroidit, filtre et concentre le filtrat par distillation sous pression réduite. Le résidu obtenu est fixé sur une colonne de gel de silice et on élue avec un mélange toluène-méthanol 87/13. On obtient ainsi 69 g de N-méthylpipérazino-2 dichloro-4,6 méthylthio-5 pyrimidine, qui fond à 80 °C.

## Exemple 29

N-méthylpipérazino-2 amino-4 méthylthio-5 chloro-6 pyrimidine

On chauffe en autoclave à 130 °C, pendant 3 heures, une solution de 30 g de N-méthylpipérazino-2 dichloro-4,6 méthylthio-5 pyrimidine dans 330 ml de méthanol contenant 60 g d'ammoniac. Puis on évapore sous pression réduite et fixe le résidu sur une colonne de gel de silice. On élue avec un mélange chloroforme-diéthylamine 98/2. On obtient ainsi 10 g de N-méthylpipérazino-2 amino-4 méthylthio-5 chloro-6 pyrimidine, qui fond à 131 °C.

## Exemple 30 (signalé pour référence aux exemples suivants)

N-méthylpipérazino-2 isopropylamino-4 méthylthio-5 chloro-6 pyrimidine

On chauffe au reflux pendant 16 heures, sous la pression atmosphérique, une solution de N-méthylpipérazino-2 dichloro-4,6 méthylthio-5 pyrimidine dans du toluène contenant de l'isopropylamine, cette dernière étant en excès (c'est-à-dire que le rapport molaire isopropylamine/N-méthylpipérazino-2 dichloro-4,6 méthylthio-5 pyrimidine est supérieur à 1). Le mélange réactionnel est ensuite traité comme indiqué à l'exemple 29. On obtient ainsi la N-méthylpipérazino-2 isopropylamino-4 méthylthio-5 chloro-6 pyrimidine, qui fond à 68 °C.

Exemple 31

N-méthylpipérazino-2 sec-butylamino-4 méthylthio-5 chloro-6 pyrimidine

On opère comme à l'exemple 30, en partant de 23,5 g de N-méthylpipérazino-2 dichloro-4,6 méthylthio-5 pyrimidine et de 25 ml de sec-butylamine (au lieu de l'isopropylamine). On obtient 20 g de N-méthylpipérazino-2 sec-butylamino-4 méthylthio-5 chloro-6 pyrimidine. Ce dernier composé, soumis à l'action de l'acide chlorhydrique gazeux au sein de l'oxyde diéthylique, donne un sel qui est mélange équimoléculaire de monochlorhydrate et de dichlorhydrate fondant à 186 °C.

Exemple 32

N-méthylpipérazino-2 tert-butylamino-4 méthylthio-5 chloro-6 pyrimidine

On chauffe au reflux pendant 28 heures une solution de 11,7 g de N-méthylpipérazino-2 dichloro-4,6 méthylthio-5 pyrimidine et 24 ml de tert-butylamine dans la méthyléthylcétone. Le mélange réactionnel est ensuite traité comme indiqué à l'exemple 29. On obtient ainsi 11,9 g de N-méthylpipérazino-2 tert-butylamino-4 méthylthio-5 chloro-6 pyrimidine, dont le chlorhydrate fond à 246 °C.

Exemple 33

N-méthylpipérazino-2 cyclopropylamino-4 méthylthio-5 chloro-6 pyrimidine

On opère comme à l'exemple 30, en partant de 11,7 g de N-méthylpipérazino-2 dichloro-4,6 méthylthio-5 pyrimidine et de 11,2 ml de cyclopropylamine (au lieu de l'isopropylamine). On obtient 9,9 g de N-méthylpipérazino-2 cyclopropylamino-4 méthylthio-5 chloro-6 pyrimidine, dont le chlorhydrate fond à 232 °C.

Exemple 34

N-méthylpipérazino-2 cyclopropylméthylamino-4 méthylthio-5 chloro-6 pyrimidine

On opère comme à l'exemple 30 en partant de 11,7 g de N-méthylpipérazino-2 dichloro-4.6 méthylthio-5 pyrimidine et de 3,1 g de cyclopropylméthylamine. On obtient 7,6 g de N-méthylpipérazino-2 cyclopropylméthylamino-4 méthylthio-5 chloro-6 pyrimidine, dont le chlorhydrate fond à 211 °C.

Exemple 35

N-méthylpipérazino-2 cyclobutylamino-4 méthylthio-5 chloro-6 pyrimidine

On opère comme à l'exemple 30 en partant de 11,7 g de N-méthylpipérazino-2 dichloro-4,6 méthylthio-5 pyrimidine et de 3,1 g de cyclobutylamine. On obtient 10,4 g de N-méthylpipérazino-2 cyclobutylamino-4 méthylthio-5 chloro-6 pyrimidine, dont le chlorhydrate fond à 226 °C.

Exemple 36

N-méthylpipérazino-2 cyclopentylamino-4 méthylthio-5 chloro-6 pyrimidine

On opère comme à l'exemple 30, en partant de 11,7 g de N-méthylpipérazino-2 dichloro-4,6 méthylthio-5 pyrimidine et de 15 ml de cyclopentylamine. On obtient 11,1 g de N-méthylpipérazino-2 cyclopentylamino-4 méthylthio-5 chloro-6 pyrimidine, dont le chlorhydrate fond à 202 °C.

Exemple 37

N-méthylpipérazino-2 cyclohexylamino-4 méthylthio-5 chloro-6 pyrimidine

On opère comme à l'exemple 30, en partant de 11,7 g de N-méthylpipérazino-2 dichloro-4,6 méthylthio-5 pyrimidine et de 5,5 ml de cyclohexylamine (au lieu de l'isopropylamine). La N-méthylpipérazino-2 cyclohexylamino-4 méthylthio-5 chloro-6 pyrimidine ainsi obtenue est transformée en son chlorhydrate par action de l'acide chlorhydrique au sein de l'oxyde diéthylique. Après deux recristallisations de ce dernier produit dans l'eau, on obtient 7,7 g de chlorhydrate de N-méthylpipérazino-2 cyclohexylamino-4 méthylthio-5 chloro-6 pyrimidine, qui fond à 202 °C.

Propriétés pharmacologiques

Affinité pour les sites récepteurs de la clonidine

Cette affinité est mesurée sur des membranes de cortex de rat selon la méthode de Greenberg D.A. et Coll., Life Sciences, *19*, 69 (1976). Elle est exprimée par une valeur $K_i$, en nanomoles (nM), qui est calculée par la formule

$$K_i = IC_{50} \left[ \frac{1}{1 + \frac{C}{K_D}} \right]$$

dans laquelle C représente la concentration de $^3H$ clonidine, $K_D$ une constante d'affinité caractéristique de la clonidine et $IC_{50}$ la concentration de produit en nonomoles nécessaire pour obtenir une inhibition de 50 % de la liaison de la $^3H$ clonidine.

Les résultats obtenus sont rassemblés dans le tableau suivant où figurent également, à titre comparatif, ceux fournis par deux produits de référence (Yohimbine, miansérine).

| Produit | $K_i$ (nM) |
|---|---|
| exemple 8 | 51 |
| " 9 | 44 |
| " 10 | 5 |
| " 12 | 51 |
| " 21 | 16 |
| " 25 | 51 |
| " 27 | 36 |
| " 30 | 26 |
| " 35 | 51 |
| " 36 | 22 |
| Yohimbine | 45 |
| Miansérine | 30 |

Propriétés toxicologiques

Les toxicités aiguës des composés de formule (I)

pour lesquels $Y_2 = Cl$ et $Y_1 =$ 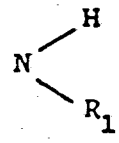

ont été déterminées chez la souris mâle $CD_1$ (Charles River) par voie orale. Les $DL_{50}$ (doses léthales 50 %) ont été calculées, après 3 jours d'observation, par la méthode cumulative de J.J. Reed et H. Muench (Amer. J. Hyg. 1938, *27*, 493).

Les composés sont relativement peu toxiques pour la souris, puisque leurs $DL_{50}$ se situent entre 200 et 1 000 mg/kg.

Utilisation thérapeutique

Les composés de formule (I) pour lesquels

$Y_2 = Cl$ et $Y_1 =$ 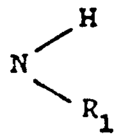

et leurs sels avec un acide pharmaceutiquement acceptable peuvent être utilisés en thérapeutique humaine, sous forme de comprimés, capsules, gélules, suppositoires, solution ingérables ou injectables, etc..., comme antihypertenseurs, antimigraineux, antidépresseurs et pour le traitement de la sénescence, de la maladie de Parkinson ou des symptômes observés lors du sevrage aux opiacés.

La posologie dépend des effets recherchés et de la voie d'administration utilisée. Par exemple, par voie orale, elle peut être comprise entre 10 et 500 mg de substance active par jour, avec des doses unitaires allant de 2 à 100 mg.

**Revendications**

1. Composés de formule générale

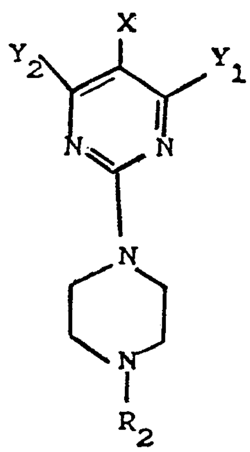

(I)

dans laquelle X est un atome d'hydrogène ou de chlore ou un groupe alkyle, alcoxy ou alkylthio ayant 1 à 3 atomes de carbone, $R_2$ est un atome d'hydrogène ou un groupe alkyle ayant 1 à 3 atomes de carbone, $Y_2$ est un atome de chlore et $Y_1$ est un atome de chlore ou un groupe

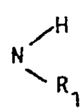

dans lequel $R_1$ est un atome d'hydrogène, un groupe alkyle à chaîne droite ou ramifiée ayant 1 à 7 atomes de carbone, un groupe cycloalkyle ayant 3 à 7 atomes de carbone ou un groupe cycloalkylalkyle ayant 4 à 8 atomes de carbone, ou bien $Y_2$ et $Y_1$ sont tous les deux des groupes OH, à l'exception de la N-méthylpipérazinyl-2 isopropylamino-4 méthylthio-5 chloro-6 pyrimidine.

2. Composés selon la revendication 1, caractérisés en ce que $Y_2$ est un atome de chlore et $Y_1$ est un groupe

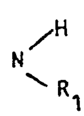

$R_1$ étant tel que défini à la revendication 1.

3. Composés selon la revendication 2, caractérisé en ce que $R_1$ est un groupe alkyle à chaîne ramifiée.

4. Composés selon la revendication 3, caractérisés en ce que $R_1$ est le groupe isopropyle.

5. Le composé pipérazino-2 isopropylamino-4 chloro-6 méthylthio-5 pyrimidine.

6. Le composé N-méthylpipérazino-2 isopropylamino-4 dichloro-5,6 pyrimidine.

7. Le composé N-méthylpipérazino-2 isopropylamino-4 chloro-6 méthoxy-5 pyrimidine.

8. Le composé pipérazino-2 isopropylamino-4 méthyl-5 chloro-6 pyrimidine.

9. Le composé N-méthylpipérazino-2 isopropylamino-4 chloro-6 méthyl-5 pyrimidine.

10. Procédé de préparation des composés selon la revendication 1 pour lesquels $Y_1$ et $Y_2$ sont des groupes OH, caractérisé en ce que l'on condense un diester de formule

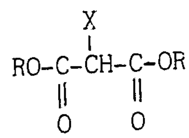

(II)

dans laquelle X a la même signification que dans la revendication 1 et R est un groupe alkyle de bas poids moléculaire, avec une amidine de formule

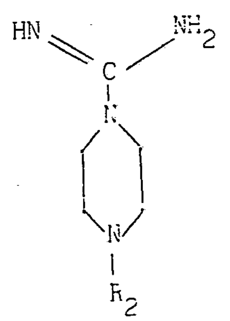

(III)

dans laquelle $R_2$ a la même signification que dans la revendication 1.

11. Procédé de préparation des composés selon la revendication 1 pour lesquels $Y_1$ et $Y_2$ sont des atomes de chlore, caractérisé en ce que l'on fait réagir un composé selon la revendication 1 pour lequel $Y_1$ et $Y_2$ sont des groupes OH avec un agent chlorurant.

12. Procédé de préparation des composés selon la revendication 1 pour lesquels $Y_1$ et $Y_2$ sont des atomes de chlore et $R_2$ est un groupe alkyle ayant 1 à 3 atomes de carbone, caractérisé en ce que l'on condense une trichloro-2,4,6 pyrimidine de formule

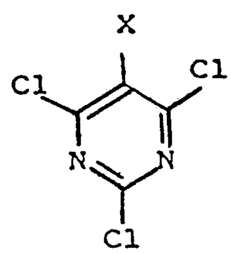

(IV)

dans laquelle X a la même signification que dans la revendication 1 avec une pipérazine de formule

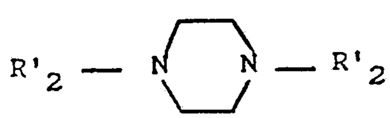

(V)

dans laquelle $R'_2$ est un groupe alkyle ayant 1 à 3 atomes de carbone.

13. Procédé de préparation des composés selon la revendication 1 pour lesquels $Y_2$ est un atome de chlore, $Y_1$ est un groupe

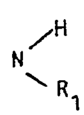

et $R_2$ est un atome d'hydrogène, caractérisé en ce que l'on hydrolyse les dérivés N-acylés de formule

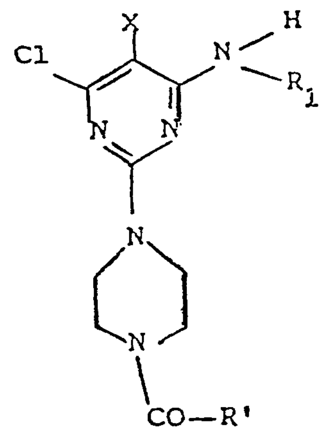

(VI)

dans laquelle X et $R_1$ ont la même signification que dans la revendication 1 et R′ est un atome d'hydrogène, un groupe alkyle de bas poids moléculaire ou le groupe phényle.

14. Procédé de préparation des composés selon la revendication 1 pour lesquels $Y_1$ est un groupe

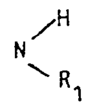

$Y_2$ est un atome de chlore et $R_2$ est un groupe alkyle, caractérisé en ce que l'on condense un composé de formule $R_1NH_2$, dans laquelle $R_1$ a la même signification que dans la revendication 1, avec un composé de formule

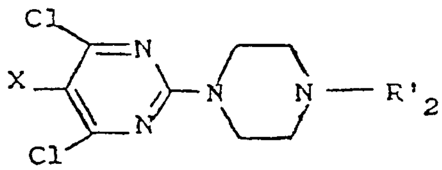

(IX)

dans laquelle X a la même signification que dans la revendication 1 et $R'_2$ est un groupe alkyle ayant 1 à 3 atomes de carbone.

15. Procédé de préparation des composés selon la revendication 1 pour lesquels $Y_1$ est un groupe

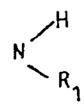

$Y_2$ est un atome de chlore et $R_2$ est un groupe alkyle, caractérisé en ce que l'on condense une pipérazine de formule

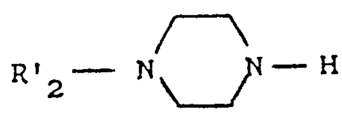

(X)

dans laquelle $R'_2$ est un groupe alkyle ayant 1 à 3 atomes de carbone, avec une dichloro-2,6 pyrimidine de formule

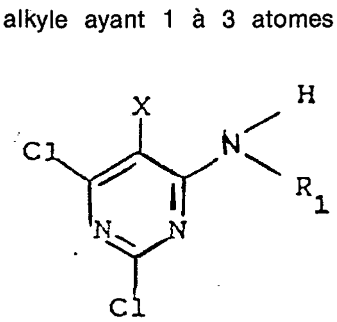

(XI)

dans laquelle X et $R_1$ ont la même signification que dans la revendication 1.

16. Médicament, particulièrement utile comme antihypertenseur, antimigraineux, antidépresseur et pour le traitement de la sénescence, de la maladie de Parkinson ou des symptômes observés lors du sevrage aux opiacés, caractérisé en ce qu'il contient, en tant que principe actif, un composé avec un acide pharmaceutiquement acceptable.

17. Médicament selon la revendication 16, caractérisé en ce qu'il contient, en tant que principe actif, le composé pipérazino-2 isopropylamino-4 chloro-6 méthylthio-5 pyrimidine ou un sel de ce composé avec un acide pharmaceutiquement acceptable.

18. Médicament selon la revendication 16, caractérisé en ce qu'il contient, en tant que principe actif, le composé N-méthylpipérazino-2 isopropylamino-4 dichloro-5,6 pyrimidine ou un sel de ce composé avec un acide pharmaceutiquement acceptable.

19. Médicament selon la revendication 16, caractérisé en ce qu'il contient, en tant que principe actif, le composé N-méthylpipérazino-2 isopropylamino-4 chloro-6 méthoxy-5 pyrimidine ou un sel de ce composé avec un acide pharmaceutiquement acceptable.

20. Médicament selon la revendication 16, caractérisé en ce qu'il contient, en tant que principe actif, le composé N-méthylpipérazino-2 isopropylamino-4 chloro-6 méthyl-5 pyrimidine ou un sel de ce composé avec un acide pharmaceutiquement acceptable.

21. Médicament selon la revendication 16, caractérisé en ce qu'il contient, en tant que principe actif, le composé pipérazino-2 isopropylamino-4 méthyl-5 chloro-6 pyrimidine.

**Claims**

1. Compounds of the general formula

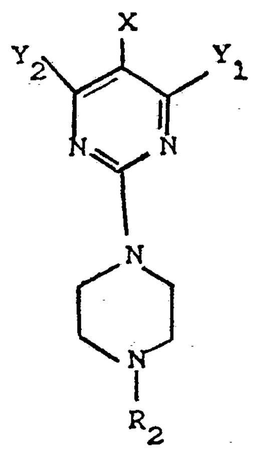

(I)

in which X is a hydrogen or chlorine atom or an alkyl, alkoxy or alkylthio group having 1 to 3 carbon atoms, $R_2$ is a hydrogen atom or an alkyl group having 1 to 3 carbon atoms, $Y_2$ is a chlorine atom and $Y_1$ is a chlorine atom or a

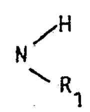

group, in which $R_1$ is a hydrogen atom, a straight or branched chain alkyl group having 1 to 7 carbon atoms, a cycloalkyl group having 3 to 7 carbon atoms or a cycloalkylalkyl group having 4 to 8 carbon atoms, or $Y_2$ and $Y_1$ are both OH groups, with the exception of 2-N-methylpiperazinyl-4-isopropylamino-5-methylthio-6-chloro-pyrimidine.

2. Compounds according to Claim 1, characterised in that $Y_2$ is a chlorine atom and $Y_1$ is a

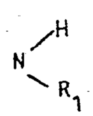

group, $R_1$ being as defined in Claim 1.

3. Compounds according to Claim 2, characterised in that $R_1$ is a branched-chain alkyl group.

4. Compounds according to Claim 3, characterised in that $R_1$ is the isopropyl group.

5. The compound 2-piperazino-4-isopropylamino-6-chloro-5-methylthio-pyrimidine.

6. The compound 2-N-methylpiperazino-4-isopropylamino-5,6-dichloro-pyrimidine.

7. The compound 2-N-methylpiperazino-4-isopropylamino-6-chloro-5-methoxy-pyrimidine.

8. The compound 2-piperazino-4-isopropylamino-5-methyl-6-chloro-pyrimidine.

9. The compound 2-N-methylpiperazino-4-isopropylamino-6-chloro-5-methyl-pyrimidine.

10. Process for the preparation of the compounds according to Claim 1 for which $Y_1$ and $Y_2$ are OH groups, characterised in that a diester of the formula

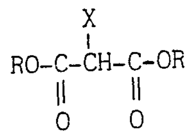

(II)

in which X has the same meaning as in Claim 1 and R is a low molecular weight alkyl group, is condensed with an amidine of the formula

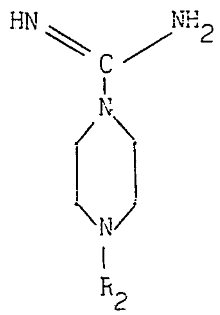

(III)

in which $R_2$ has the same meaning as in Claim 1.

11. Process for the preparation of the compounds according to Claim 1 for which $Y_1$ and $Y_2$ are chlorine atoms, characterised in that a compound according to Claim 1 for which $Y_1$ and $Y_2$ are OH groups is reacted with a chlorinating agent.

12. Process for the preparation of the compounds according to Claim 1 for which $Y_1$ and $Y_2$ are chlorine atoms and $R_2$ is an alkyl group having 1 to 3 carbon atoms, characterised in that a 2,4,6-trichloro-pyrimidine of the formula :

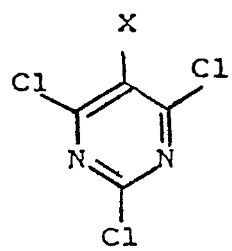

(IV)

in which X has the same meaning as in Claim 1 is condensed with a piperazine of the formula

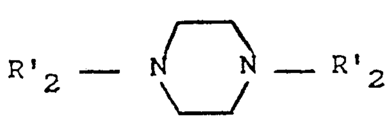

(V)

in which $R'_2$ is an alkyl group having 1 to 3 carbon atoms.

13. Process for the preparation of the compounds according to Claim 1 for which $Y_2$ is a chlorine atom, $Y_1$ is a

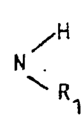

group and $R_2$ is a hydrogen atom, characterised in that the N-acylated derivatives of the formula

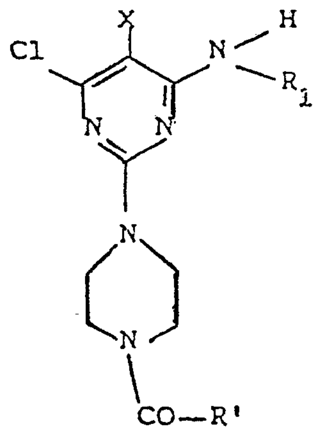

(VI)

in which X and $R_1$ have the same meaning as in Claim 1 and R' is a hydrogen atom, a low molecular weight alkyl group or the phenyl group, are hydrolysed.

14. Process for the preparation of the compounds according to Claim 1 for which $Y_1$ is

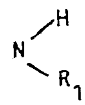

$Y_2$ is a chlorine atom and $R_2$ is an alkyl group, characterised in that a compound of the formula $R_1NH_2$, in which $R_1$ has the same meaning as in Claim 1, is condensed with a compound of the formula

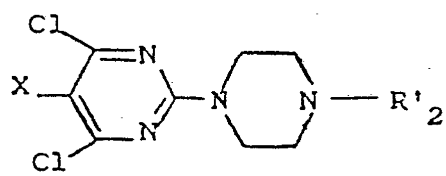

(IX)

in which X has the same meaning as in Claim 1 and $R'_2$ is an alkyl group having 1 to 3 carbon atoms.

15. Process for the preparation of the compounds according to Claim 1 for which $Y_1$ is a

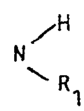

$Y_2$ is a chlorine atom and $R_2$ is an alkyl group, characterised in that the piperazine of the formula

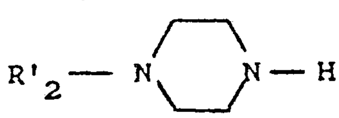

(X)

in which $R'_2$ is an alkyl group having 1 to 3 carbon atoms in condensed with one 2,6-dichloropyrimidine of the formula

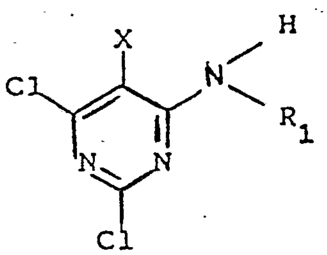

(XI)

in which X and $R_1$ have the same meaning as in Claim 1.

16. Medicament, especially useful as an anti-hypertensive, anti-migraine and anti-migraine and anti-depressive agent and for the treatment of senescence, Parkinson's disease or symptoms observed upon withdrawal of opiates, characterised in that it contains, as the active principle, a compound according to Claim 2 or a salt of such a compound with a pharmamceutically acceptable acid.

17. Medicament according to Claim 16, characterised in that it contains, as the active principle, the compound 2-piperazino-4-isopropylamino-6-chloro-5-methylthio-pyrimidine or a salt of this compound with a pharmaceutically acceptable acid.

18. Medicament according to Claim 16, characterised in that it contains, as the active principle, the compound 2-N-methylpiperazino-4-isopropylamino-5,6-dichloro-pyrimidine or a salt of this compound with a pharmaceutically acceptable acid.

19. Medicament according to Claim 16, characterised in that it contains, as the active principle, the compound 2-N-methylpiperazino-4-isopropylamino-6-chloro-5-methoxy-pyrimidine or a salt of this compound with a pharmaceutically acceptable acid.

20. Medicament according to Claim 16, characterised in that it contains, as the active principle, the compound 2-N-methylpiperazino-4-isopropylamino-6-chloro-5-methyl-pyrimidine or a salt of this compound with a pharmaceutically acceptable acid.

21. Medicament according to Claim 16, characterised in that it contains, as the active principle, the compound 2-piperazino-4-isopropylamino-5-methyl-6-chloro-pyrimidine.

**Ansprüche**

1. Verbindungen der allgemeinen Formel (I)

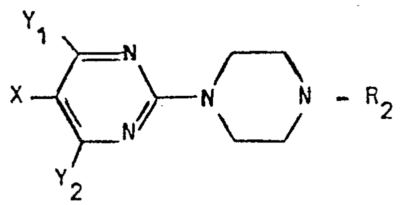

(I)

worin X ein Wasserstoff- oder Chloratom oder eine Alkyl-, Alkoxy- oder Alkyklthiogruppe mit 1 bis 3 Kohlenstoffatomen ist, $R_2$ ein Wasserstoffatom oder eine Alkylgruppe mit 1 bis 3 Kohlenstoffatomen darstellt, $Y_2$ ein Chloratom ist und $Y_1$ ein Chloratom oder eine Gruppe

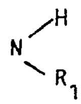

darstellt, worin $R_1$ ein Wasserstoffatom, eine gerad- oder verzweigtkettige Alkylgruppe mit 1 bis 7 Kohlenstoffatomen, eine Cycloalkylgruppe mit 3 bis 7 Kohlenstoffatomen oder eine Cycloalkylalkylgruppe mit 4 bis 8 Kohlenstoffatomen ist, oder aber $Y_2$ und $Y_1$ sind beide OH-Gruppen mit der Ausnahme von 2-N-Methylpiperazinyl-4-isopropylamino-5-methylthio-6-chlor-pyrimidin.

2. Verbindungen gemäß Anspruch 1, dadurch gekennzeichnet, daß $Y_2$ ein Chloratom und $Y_1$ eine Gruppe

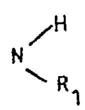

ist, worin $R_1$ wie in Anspruch 1 definiert ist.

3. Verbindungen gemäß Anspruch 2, dadurch gekennzeichnet, daß $R_1$ eine Alkylgruppe mit verzweigter Kette ist.

4. Verbindungen gemäß Anspruch 3, dadurch gekennzeichnet, daß $R_1$ die Isopropylgruppe ist.

5. Die Verbindung 2-Piperazino-4-isopropylamino-6-chlor-5-methylthio-pyrimidin.

6. Die Verbindung 2-N-Methylpiperazino-4-isopropylamino-5,6-dichlorpyrimidin.

7. Die Verbindung 2-N-Methylpiperazino-4-isopropylamino-6-chlor-5-methoxypyrimidin.

8. Die Verbindung 2-Piperazino-4-isopropylamino-5-methyl-6-chlorpyrimidin.

9. Die Verbindung 2-N-Methylpiperazino-4-isopropylamino-6-chlor-5-methylpyrimidin.

10. Verfahren zur Herstellung der Verbindungen gemäß Anspruch 1, für die $Y_1$ und $Y_2$ OH-Gruppen bedeuten, dadurch gekennzeichnet, daß man einen Diester der allgemeinen Formel (II)

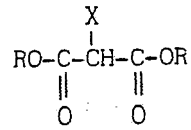

(II)

worin X dieselbe Bedeutung wie in Anspruch 1 hat und R eine Alkylgruppe mit niedrigem Molekulargewicht ist, mit einem Amidin der Formel (III)

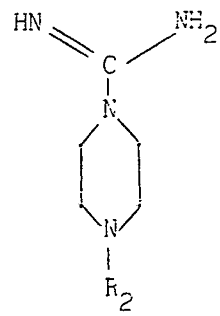

(III)

worin $R_2$ dieselbe Bedeutung wie in Anspruch 1 hat, kondensiert.

11. Verfahren zur Herstellung der Verbindungen gemäß Anspruch 1, für die $Y_1$ und $Y_2$ Chloratome bedeuten, dadurch gekennzeichnet, daß man eine Verbindung gemäß Anspruch 1, für die $Y_1$ und $Y_2$ OH-Gruppen bedeuten, mit einem chlorierenden Mittel zur Reaktion bringt.

12. Verfahren zur Herstellung der Verbindungen gemäß Anspruch 1, für die $Y_1$ und $Y_2$ Chloratome bedeuten und $R_2$ eine Alkylgruppe mit 1 bis 3 Kohlenstoffatomen ist, dadurch gekennzeichnet, daß man ein 2,4,6-Trichlorpyrimidin der Formel (IV)

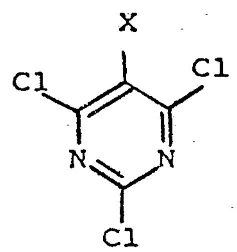

(IV)

worin X dieselbe Bedeutung wie in Anspruch 1 hat, mit einem Piperazin der Formel (V)

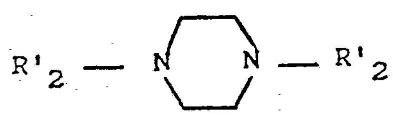

(V)

worin $R'_2$ eine Alkylgruppe mit 1 bis 3 Kohlenstoffatomen ist, kondensiert.

13. Verfahren zur Herstellung der Verbindungen gemäß Anspruch 1, für die $Y_2$ ein Chloratom ist, $Y_1$ eine Gruppe

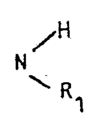

bedeutet und $R_2$ ein Wasserstoffatom ist, dadurch gekennzeichnet, daß man die N-acylierten Derivate der Formel (VI)

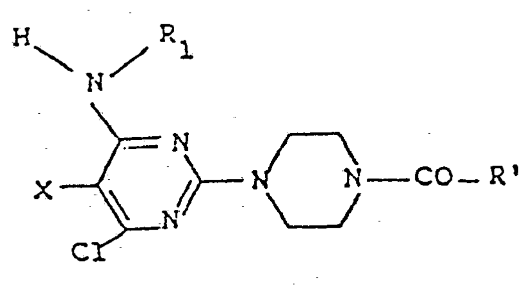

(VI)

worin X und $R_1$ dieselbe Bedeutung wie in Anspruch 1 haben und R' ein Wasserstoffatom, eine Alkylgruppe mit niedrigem Molekulargewicht oder die Phenylgruppe ist, hydrolysiert.

14. Verfahren zur Herstellung der Verbindungen gemäß Anspruch 1, für die $Y_1$ eine

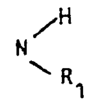

Gruppe bedeutet, $Y_2$ ein Chloratom ist und $R_2$ eine Alkylgruppe bedeutet, dadurch gekennzeichnet, daß man eine Verbindung der Formel $R_1 NH_2$, worin $R_1$ dieselbe Bedeutung wie in Anspruch 1 hat, mit einer Verbindung der Formel (IX)

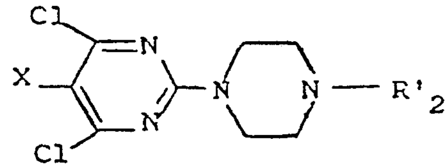

(IX)

worin X dieselbe Bedeutung wie in Anspruch 1 hat und $R'_2$ eine Alkylgruppe mit 1 bis 3 Kohlenstoffatomen bedeutet, kondensiert.

15. Verfahren zur Herstellung der Verbindungen gemäß Anspruch 1, für die $Y_1$ eine Gruppe

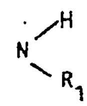

bedeutet, $Y_2$ ein Chloratom ist und $R_2$ eine Alkylgruppe bedeutet, dadurch gekennzeichnet, daß man ein Piperazin der Formel (X)

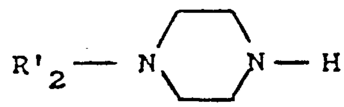

(X)

worin $R'_2$ eine Alkylgruppe mit 1 bis 3 Kohlenstoffatomen ist, mit einem 2,6-Dichlorpyrimidin der Formel (XI)

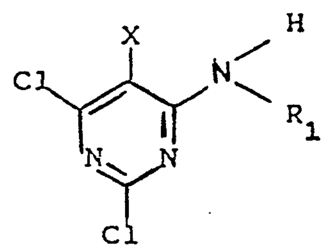

(XI)

worin X und $R_1$ dieselbe Bedeutung wie in Anspruch 1 haben, kondensiert.

16. Arzneimittel besonders nützlich als Antihypertensionsmittel, Antimigränemittel, antidepressorisches Mittel und zur Behandlung der Seneszenz, der Parkinson'schen Krankheit oder der Symptome, die bei der Entziehung an Rauschgiftsüchtigen beobachtet werden, dadurch gekennzeichnet, daß es als aktives Prinzip eine Verbindung gemäß Anspruch 2 oder ein Salz einer solchen Verbindung mit einer pharmazeutisch annehmbaren Säure enthält.

17. Arzneimittel gemäß Anspruch 16, dadurch gekennzeichnet, daß es als aktives Prinzip die Verbindung 2-Piperazino-4-isopropylamino-6-chlor-5-methylthiopyrimidin oder ein Salz dieser Verbindung mit einer pharmazeutisch annehmbaren Säure enthält.

18. Arzneimittel gemäß Anspruch 16, dadurch gekennzeichnet, daß es als aktives Prinzip die Verbindung 2-N-Methylpiperazino-4-isopropylamino-5,6-dichlorpyrimidin oder ein Salz dieser Verbindung mit einer pharmazeutisch annehmbaren Säure enthält.

19. Arzneimittel gemäß Anspruch 16, dadurch gekennzeichnet, daß es als aktives Prinzip die Verbindung 2-N-Methylpiperazino-4-isopropylamino-6-chlor-5-methoxypyrimidin oder ein Salz dieser Verbindung mit einer pharmazeutisch annehmbaren Säure enthält.

20. Arzneimittel gemäß Anspruch 16, dadurch gekennzeichnet, daß es als aktives Prinzip die Verbindung 2-N-Methylpiperazino-4-isopropylamino-6-chlor-5-methylpyrimidin oder ein Salz dieser Verbindung mit einer pharmazeutisch annehmbaren Säure enthält.

21. Arzneimittel gemäß Anspruch 16, dadurch gekennzeichnet, daß es als aktives Prinzip die Verbindung 2-Piperazino-4-isopropylamino-5-methyl-6-chlorpyrimidin enthält.